# EUROPEAN PATENT APPLICATION

(11) **EP 4 295 885 A1**
(43) Date of publication of application: **27.12.2023**
(21) Application number: 21926271.4
(22) Date of filing: 30.07.2021
(51) Int. Cl.: A61M 25/00, A61M 25/10, A61B 17/02

(54) **BALLOON CATHETER RETRACTOR**

(30) Priority: 19.02.2021 CN 202110190768
(71) Applicant: Shanghai Keci Medical Technology Co., Ltd, Shanghai 201613 (CN)
(72) Inventor: ZHANG, Zhichao, Shanghai 200135 (CN); MA, Changsheng, Shanghai 200135 (CN); DONG, Jianzeng, Shanghai 200135 (CN)
(74) Representative: Cameron Intellectual Property Ltd
(86) International application number: PCT/CN2021/109615
(87) International publication number: WO 2022/174557

(57) **Abstract**

A balloon catheter retractor (1), comprising a balloon catheter (13) and a balloon (12) fixed on the balloon catheter (13), wherein when the balloon (12) is inflated with a fluid, the balloon (12) bends and drives the balloon catheter (13) to bend correspondingly. By applying a radial limit to the balloon (12), the balloon (12) does not expand in a diametrical direction when expanding to a certain diameter. The balloon catheter retractor (1) can enhance the bending capacity and the characteristic of maintaining the bending performance.

## Description

### TECHNICAL FIELD

The present invention relates to the field of medical devices, in particular a balloon catheter traction device.

### BACKGROUND

In the prior art, the scheme for retracting the esophagus by using the balloon has the following two solutions:

The balloon is manufactured to create a C-shaped structure for dilation by using a non-compliant material. Before use, the residual air is removed from the balloon by suction, and a rigid guidewire is inserted through the balloon's axis to straighten it from its collapsed C-shape to a straight line. During the procedure, the balloon is inserted into the esophageal. After withdrawing the rigid guidewire and introducing liquid while maintaining a certain pressure (greater than 4 atmospheres), the balloon regains its preformed C-shaped structure, providing support for traction. However, due to the use of non-compliant balloons, the initial diameter of the balloon is relatively large, usually exceeding 8 millimeters. Additionally, embedding a rigid guidewire is necessary to facilitate its passage through the lumen. Consequently, this approach is only feasible through the oral cavity and cannot be used to access the esophageal lumen through the nasal passage. Furthermore, since it must be introduced through the oral cavity, it significantly impacts patient comfort and can only be employed on patients under general anesthesia (unconscious), making it unsuitable for awake patients.

Another type of traction device involves the use of semi-compliant balloons, characterized by an eccentric axis and localized reinforcement along the balloon's axis. As the balloon inflates, the expansion on either side of the balloon's axis is uneven, causing the balloon to bend. Ultimately, as the balloon continues to inflate, it forms a C-shaped structure to achieve the goal of deviating the esophagus. However, in this approach, as the balloon inflates, its material becomes thinner, causing the traction force of the balloon to first increase and then decrease. Additionally, the radial expansion of the balloon hinders its axial expansion, resulting in a reduced difference in expansion rates along the bending direction. This weakens the bending effect. Consequently, in this scenario, the traction force of the balloon decreases over time. During actual usage, the bending force cannot be maintained, thus failing to meet clinical requirements.

### SUMMARY

This application addresses the issues of significant patient discomfort and unsustainable bending force caused by existing balloon catheter retractor or traction devices. A balloon catheter traction device is provided that enhances its bending capability and improves its ability to maintain its bending performance.

In order to achieve this objective, the present invention provides a balloon catheter traction device comprising a balloon catheter and a balloon fixed onto the balloon catheter. When fluid is inflated into the balloon, it bends and subsequently causes the balloon catheter to bend accordingly. When a radial restraint or radial limiting feature is applied to the balloon, it prevents further expansion in the radial direction once the balloon reaches a certain diameter.

In this context, the balloon is manufactured with semi-compliant characteristics, resulting in an eccentrically positioned balloon concerning or with respect to the axis. The balloon features a fixed-length restraint on the inner side in the axial intended bending direction, without any restriction on the outer side in the axial intended bending direction. The fixed-length restraint on the inner side of the balloon's axial intended bending direction could be reinforced ribs positioned on the inner side of the balloon's axial bending direction. The fixed-length restraint on the inner side of the balloon's axial intended bending direction could be lines fixed at both ends of the balloon. The radial restraint of the balloon could be achieved by using a mesh or sleeve with a fixed or limited diameter, or even by utilizing another balloon with a fixed diameter. The radial restraint of the balloon could also be achieved by modifying specific regions of the balloon to increase the modulus and stiffness of those regions.

An optimal approach for radial restraint or limiting of the balloon is a woven mesh around the outer circumference of the balloon. This woven mesh exhibits different stretching properties in the radial and axial directions. The longitudinal threads of the mesh possess a strong elastic modulus that resists radial expansion and maintains a fixed diameter. Meanwhile, the latitudinal threads of the woven mesh offer elasticity, enabling the mesh to have stretching characteristics in the axial direction. The weaving method or pattern of the mesh involves denser longitudinal threads and sparser latitudinal threads. The two ends of the woven mesh are fixed to the two ends of the balloon. The mesh is initially in a folded and rolled configuration along with the balloon. During use, as the balloon on the balloon catheter inflates and bends, the woven mesh follows the bending. Once the diameter of the balloon reaches the preset diameter of the woven mesh, even if the balloon is further inflated, its diameter no longer changes. The latitudinal threads of the woven mesh consist of multiple segments of short threads, resulting in a structure of segmented woven mesh. The segmented woven mesh features several complete latitudinal threads on the inner side of the balloon's axial intended bending direction, acting as fixed-length restraints. The woven mesh has one or a few nearby latitudinal threads replaced with longitudinal threads of the same material on the inner side of the balloon's anticipated axial bending direction. The material used for the balloon is an anisotropic material, with a lower elastic modulus in the axial direction and a higher elastic modulus in the radial direction. After the formation of the balloon, a series of radial reinforcement ribs or a reinforcement mesh is applied externally. A polymer membrane is applied externally to the radial reinforcement ribs or reinforcement mesh to secure them in place or for fixing them with respect to the balloon.

Furthermore, the radial restraint of the balloon involves embedding a woven mesh within the balloon. Both the inner and outer sides of the balloon are made of thermoplastic elastomeric material, encapsulating the woven mesh.

Additionally, the radial restraint of the balloon involves the external sheathing of the balloon with a memory metal mesh possessing a memory of a contracted or predetermined shape. The diameter of the memory metal mesh contracts back to its original state or shape. In the axial intended bending direction of the balloon, the memory metal mesh maintains at least one fixed-length memory metal wire on the inner side.

The advantageous effect of this device is the addition of a radial restraint to the balloon while allowing unrestricted axial expansion. This ensures that the balloon no longer expands in the radial direction once it reaches a predetermined diameter. As a result, the balloon's expansion tendency is concentrated more in the axial direction, effectively increasing the balloon's bending curvature and stiffness. The significance of this lies in significantly enhancing the balloon's bending capability, and over time, the bending force of the balloon does not diminish, ensuring the effectiveness of esophageal traction with the balloon.

To make the aforementioned objectives, features, and advantages more clearly understandable, the following text provides a detailed explanation of a preferred embodiment, accompanied by the attached diagrams:

### DESCRIPTION OF THE DRAWINGS

In order to provide a clearer understanding of the specific embodiments of the present invention or the technical solutions in the prior art, a brief introduction to the accompanying drawings required for the description of specific embodiments or existing techniques is provided below. It is evident that the accompanying drawings in the following description depict certain embodiments of the present invention. Those skilled in the art will recognize that, without departing from the spirit of this invention, additional variations can be derived based on these drawings without the necessity for inventive effort.
Figure 1 is a schematic diagram of an embodiment of the present device.
Figure 2 is a cross-sectional view of an embodiment of the present device.
Figure 3 is an enlarged schematic diagram depicting a portion of the woven mesh in an embodiment of the present device.
Figure 4 is a schematic diagram illustrating a second embodiment of the present device.
Figure 5 is an enlarged schematic diagram depicting the form of the woven mesh in the second embodiment of the present device.
Figure 6 is a schematic diagram of a third embodiment of the present device.
Figure 7 is a schematic diagram of a fourth embodiment of the present device.
Figure 8 is a cross-sectional diagram of a fourth embodiment of the present device.
Figure 9 is a schematic diagram of a fifth embodiment of the present device.
Figure 10 is a schematic diagram illustrating the memory metal mesh of the present device.

### DETAILED DESCRIPTION OF SPECIFIC EMBODIMENTS

The technical solutions in the embodiments of the present device will be clearly and completely described below in conjunction with the accompanying drawings. Obviously, the embodiments described are only a portion of the embodiments of the present device and not all of them. The components of embodiments of the present device generally described and illustrated in the accompanying drawings herein may be arranged and designed in a variety of different configurations. Accordingly, the following detailed description of embodiments provided in the accompanying drawings is not intended to limit the scope of the present device for which protection is claimed, but rather represents only selected embodiments of the present device. Based on the embodiments of the present device, all other embodiments obtained by a person skilled in the art without creative labor are within the scope of protection of the present application.

The solution of this device involves adding a radial constraint to the balloon, without imposing any restrictions axially. This prevents the balloon from expanding in the radial direction when it reaches a predetermined diameter, focusing the expansion of the balloon more on or in its axial direction. This effectively increases the curvature and bending stiffness of the balloon.

One approach is as follows: use a semi-compliant balloon to create an eccentrically positioned balloon relative to the axis. Subsequently, a diameter restriction is applied to the balloon in the radial direction. As the balloon inflates to the intended final diameter, expansion in the radial direction ceases, while expansion along the axial direction remains unrestricted, allowing the balloon to maintain continuous curvature. Over time, this approach enables the balloon to retain its curvature and bending strength.

Another approach is as follows: first use a semi-compliant balloon to create an eccentrically positioned balloon relative to the axis. Then, impose a restriction on the diameter of the balloon in the radial direction, while applying a fixed-length constraint on the inner side in the axial intended bending direction, without any restriction on the outer side in the axial intended bending direction. Consequently, as the balloon's diameter increases to the predetermined size, the balloon's diameter is restricted while reinforcing the limitation on the length of the inner side in the bending direction. This achieves the objective of enhancing bending force. At the same time, the length on the inner side in the axial intended bending direction is subjected to intensified restrictions, thereby achieving the aim of enhancing bending force.

The aforementioned diameter restriction on the balloon can involve using a mesh, sleeve, or another balloon with a fixed diameter. Alternatively, it can involve modifying certain regions of the balloon to enhance the modulus and stiffness of those areas. The axial restriction on the balloon can take the form of a reinforcing strip, a line or thread or strand affixed to the balloon, or a line or thread or strand secured at both ends of the balloon.

The following provides further detailed explanation of this invention, in order to enable professionals in the field to implement it based on the provided description.

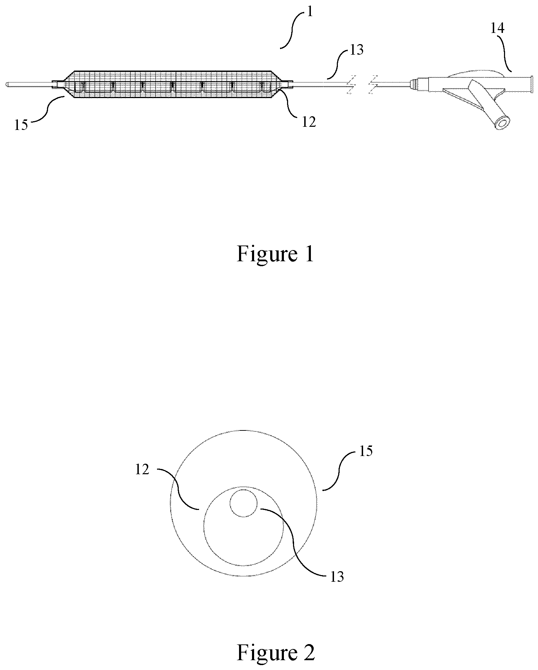

First, please refer to Figure 1, which is a schematic diagram of an embodiment of the present device. Figure 1 illustrates a balloon catheter retractor 1, featuring an eccentric balloon 12 at its center, surrounded by a woven mesh 15. The woven mesh 15 is formed by adjusting the properties of materials along the warp and weft yarns (such as elastic modulus and elongation at break) and the weaving method or pattern (tightness or looseness of the warp and weft yarns), thus creating differential stretching characteristics in the radial and axial directions of the woven mesh tube.

Next, please refer to Figure 2. Figure 2 shows a cross-sectional view of an embodiment of the present device. In Figure 2, the embodiment consists of three layers from the innermost to the outermost: the innermost layer is the balloon catheter 13, followed by the balloon 12 fitted over the outer surface of the catheter 13, and finally the outermost layer is the woven mesh 15 covering the balloon 12.

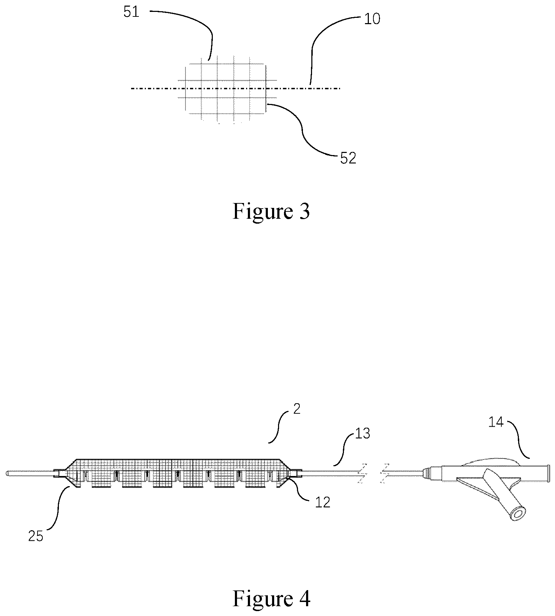

Furthermore, please refer to Figure 3. Figure 3 provides an enlarged schematic of a localized portion of the woven mesh in an embodiment of the present device. Lines or threads or strands parallel to the axis 10 of the balloon catheter are defined as the warp threads 51 of the woven mesh, while lines perpendicular to it are the weft threads 52. The warp threads 52 of the woven mesh possess high modulus and are less stretchable to maintain a fixed diameter. The weft threads 51 of the woven mesh exhibit good elasticity, characterized by their stretchability along the length direction.

The two ends of the woven mesh 15 are fastened to the two ends of the balloon 12. Simultaneously, the method or process of expelling fluid from within the balloon 12 and then folding and rolling up the mesh and balloon is employed to establish the folded and rolled configuration of the mesh and balloon as the initial state. By doing so, it ensures that the balloon catheter 13 can meet the transportability requirements of the balloon within the delivery channel before use. In the meantime, during usage, as the balloon within the balloon catheter 13 expands and bends, the woven mesh follows the curvature. Once the diameter of the balloon reaches the predetermined diameter of the woven mesh, even if the balloon is further inflated, the diameter of the balloon remains constant, effectively constraining the balloon from expanding further in the radial direction. This reinforcement enhances the balloon's elongation along the axial direction.

Figure 4 illustrates a schematic of a second embodiment of the present device. In Figure 4, a balloon catheter traction device 2 is depicted, featuring an eccentrically positioned central balloon 12 and an outer layer formed by a woven mesh 25.

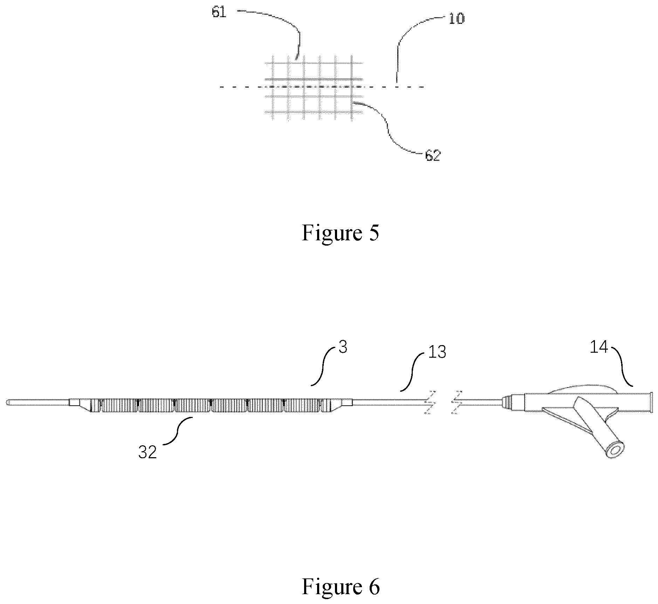

Figure 5 illustrates an enlarged schematic of the woven mesh in the second embodiment of the present device. In Figure 5, the enlarged form of the woven mesh 25 is displayed. Lines parallel to the axis 10 of the balloon catheter are designated as the warp threads 61 of the woven mesh, while lines perpendicular to it are the weft threads 62. The warp threads 62 of the woven mesh have a strong modulus, making them less stretchable and maintaining a fixed diameter. The weft threads are comprised of multiple short segments, resulting in a segmented structure for the woven mesh.

As the balloon 12 inflates and bends, the stretching side of the balloon, due to the segmented weft threads, is not constrained by the entire length of a single weft thread. This allows it to freely expand along the axial direction. Meanwhile, in the radial direction, the inflation of the balloon encounters the warp threads 61 of the woven mesh 25, imposing radial limitations. Consequently, this imparts greater deformation to the axial expansion of the balloon, enabling it to maintain curvature and stiffness.

Figure 6 presents a schematic of a third embodiment of the present device. In Figure 6, a balloon catheter traction device 3 is shown, featuring a radially reinforced balloon 32 at its center. This balloon 32 is characterized by its expansion in the radial direction, which ceases upon reaching a predetermined diameter, while allowing unrestricted free expansion along the axial direction. Specific solutions include: the balloon 32 employs an anisotropic material, characterized by a lower elastic modulus in the axial direction and a higher elastic modulus in the radial direction. Alternatively, a series of radial reinforcements or a reinforcing mesh is applied to the outer surface of the balloon 32 after its formation, followed by encapsulation with a polymer membrane on the exterior to secure the reinforcements, resulting in a smooth external surface with relatively fixed internal reinforcements or mesh.

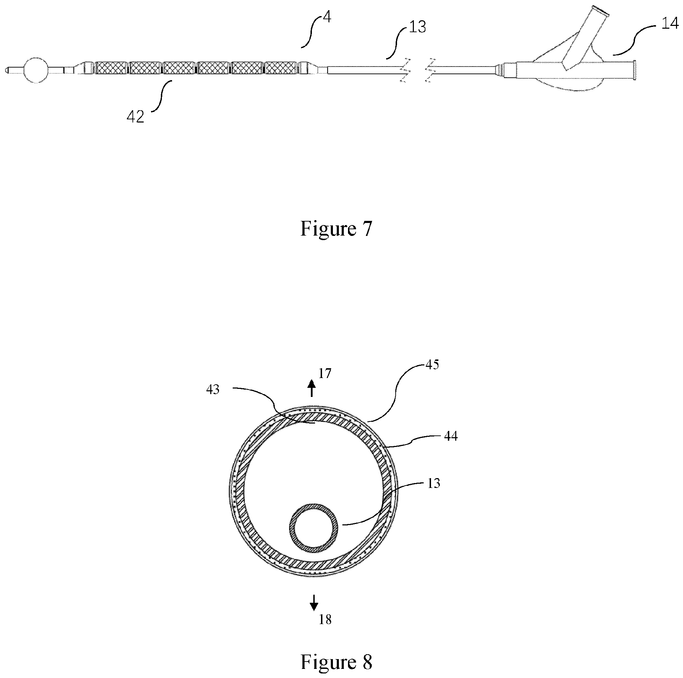

Figure 7 displays a schematic of a fourth embodiment of the present device. In Figure 7, a balloon catheter traction device 4 is depicted, featuring a balloon 42 with an embedded reinforcing woven mesh at its center. This balloon 42 is characterized by a slight contraction along the axial direction during its expansion in the radial direction. Once it inflates in the radial direction to the expected diameter, its form becomes fixed, preventing further radial expansion. This transition allows the entire balloon to expand along the axial direction. The specific structure is illustrated in Figure 8, which presents a cross-sectional view of the fourth embodiment of the present device. In Figure 8, the balloon 42 is divided into the inner balloon layer 43, an intermediate woven mesh 44, and the outer balloon layer 45. Both the inner 43 and outer 45 layers of the balloon consist of thermoplastic elastomeric material and encapsulate the woven mesh 44. As the balloon expands radially, the woven mesh 44 undergoes deformation. However, due to the presence of the thermoplastic elastomeric material, the deformation of the woven mesh 44 reaches a limit between its grid points, preventing radial expansion and allowing continued axial expansion. Throughout the deformation process, the shapes of the mesh's grids tend to change, but the underlying polymer material within the mesh tends to retain its original shape. This interaction results in a counteracting effect, limiting the extent of deformation in the woven mesh.

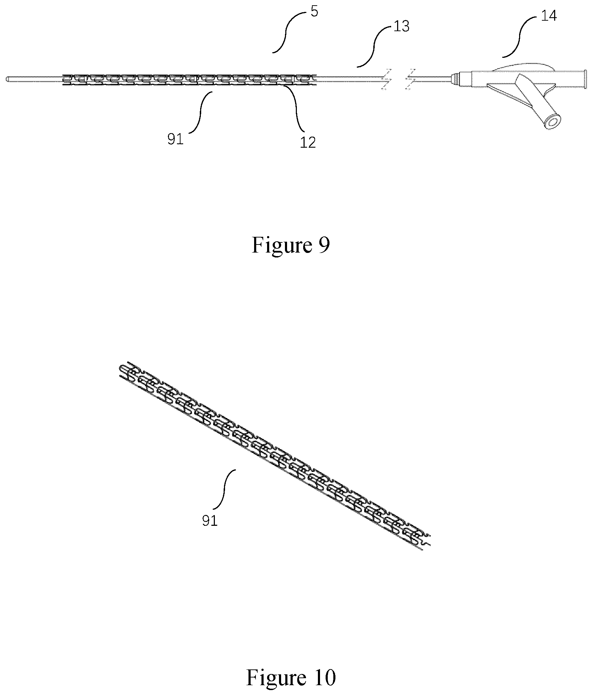

Figure 9 represents a schematic of the fifth embodiment of the present device. In this depiction, a balloon catheter traction device 5 is presented. The centerpiece of this device is an eccentrically positioned central balloon 12. Enveloping the balloon is a memory metal mesh 91, notable for its memory characteristic of a contracted shape. As the balloon 12 inflates, its diameter expands while adhering to the surface of the balloon, but its length remains unaltered. As the memory metal mesh 91 undergoes deformation, its radially positioned rings ultimately straighten into circular rings, thus constraining the expansion of the balloon's diameter. The radial length of the memory metal mesh does not change during this process. Simultaneously, on the side where the balloon elongates, there are no metal wires to restrict its extension. Therefore, after the balloon 12 bends, its diameter is constrained by the memory metal mesh 91, while its curvature remains unconstrained. Consequently, this design achieves the dual objectives of enhancing bending force and maintaining the curved shape of the balloon. Another advantage of this approach is that as the balloon 12 contracts, the memory metal mesh 91, due to its memory properties, shrinks back to its original state, effectively enveloping the balloon catheter. This reduces the outer diameter of the device after contraction, facilitating its passage during withdrawal.

A further enhancement option in this device is to add a fixed-length strut or rib on the inner side of the expected bending direction of the balloon in the above-mentioned design. This would introduce a fixed-length constraint to the inner side of the balloon in the intended axial bending direction.

Referring back to Figures 1 to 3 for the first balloon catheter traction device design, in this approach, one or several of the weft threads 51 on the inner side 18 of the expected axial bending direction of the balloon 12 can be replaced with the same material as the warp threads 52. This ensures that the length of the mesh on the inner side 18 of the expected axial bending direction remains unchanged, thus enhancing the curvature effect of the balloon from the exterior.

As the balloon inflates, there is significant friction between the balloon and the woven mesh 15, creating a relatively fixed relationship. As the balloon bends, the length of the inner side 18 in the expected axial bending direction is fixed both by the balloon catheter 13 and the warp threads on the inner side 18 in the expected axial bending direction. This ensures that the difference in length between the inner side 18 in the expected, anticipate or intended axial bending direction and the elongation side 17 does not decrease over time, thereby maintaining the ability to retain the curvature and bending force of the balloon catheter 1.

Referring back to Figures 4 and 5, in this approach, several of the weft threads 51 on the inner side 18 of the expected axial bending direction of the segmented woven mesh 25 can be reinforced. This results in a characteristic where the inner side 18 in the expected axial bending direction is less prone to elongation, thereby enhancing the curvature effect of the balloon. By reinforcing specific weft threads on the inner side 18 of the expected axial bending direction, the axial elongation of that side can be controlled, ensuring that the curvature of the balloon remains pronounced during bending. This reinforcement strengthens the curvature performance of the balloon catheter.

Referring back to Figures 9 and 10, where Figure 10 represents a schematic of the memory metal mesh in this device. In this approach, due to the inherently limited extension of memory metal in the linear direction compared to polymer materials, keeping at least one fixed-length memory metal wire on the inner side 18 in the expected axial bending direction is sufficient to render the inner side 18 in the expected, anticipated or intended axial bending direction of the balloon catheter 13 non-extensible during bending. Simultaneously, the extensibility on the elongation side 17 is retained, achieving a stronger curvature effect.

While the embodiments of the present retractor device have been disclosed as above, they are not limited to the specifications and embodiments provided. They can be applied to various fields suitable for the present device. Those skilled in the art can easily make additional modifications. Therefore, within the general concept defined by the claims and their equivalents, the present device is not limited to specific details and illustrations shown and described here.

## Claims

1. The balloon catheter traction device comprises a balloon catheter and a balloon fixed onto the balloon catheter, and when fluid is inflated into the balloon, the balloon bends and causes the balloon catheter to bend correspondingly, and a radial restraint is applied to the balloon, preventing it from further expanding in the radial direction once it reaches a certain diameter.

2. The balloon catheter traction device as in claim 1 wherein the balloon fixed onto the balloon catheter is an eccentrically positioned balloon and a semi-compliant balloon.

3. The balloon catheter traction device as in claim 1, wherein the balloon fixed onto the balloon catheter is provided with a fixed-length restraint on an inner side of an axial intended bending direction, without any restriction on an outer side of the axial intended bending direction.

4. The balloon catheter traction device as in claim 3, wherein the fixed-length restraint on the inner side of the balloon's axial intended bending direction is a reinforcing rib positioned on the inner side of the balloon's axial intended bending direction.

5. The balloon catheter traction device as in claim 3, wherein the fixed-length restraint on the inner side of the balloon's axial intended bending direction are lines or threads affixed to the inner side of the balloon's bending direction.

6. The balloon catheter traction device as in claim 3, wherein the fixed-length restraint on the inner side of the balloon's axial intended bending direction are lines or threads or strands fixed at both ends of the balloon.

7. The balloon catheter traction device as in claim 1, wherein the radial restraint of the balloon is a mesh or a sleeve with a fixed diameter, or another balloon with a fixed diameter.

8. The balloon catheter traction device as in claim 1, wherein the radial restraint of the balloon comprises specific regions of the balloon modified to increase modulus and stiffness of the balloon in those specific regions.

9. The balloon catheter traction device as in claim 1, wherein the radial restraint of the balloon is a woven mesh around the outer circumference of the balloon, and the woven mesh exhibits different stretching properties in the radial and axial directions.

10. The balloon catheter traction device as in claim 9, wherein longitudinal threads of the woven mesh possess a strong elastic modulus, resisting stretching and maintaining a fixed diameter, and the latitudinal threads of the woven mesh possess good elasticity, enabling the mesh to have stretching characteristics in the axial direction.

11. The balloon catheter traction device as in claim 9, wherein a weaving method or pattern of the woven mesh provides denser longitudinal threads and sparser latitudinal threads.

12. The balloon catheter traction device as in claim 9, wherein ends of the woven mesh are fixed at both ends of the balloon, and the woven mesh and balloon are initially in a folded and rolled configuration, and during use, as the balloon inside the balloon catheter inflates and bends, the woven mesh follows the bending, and once a diameter of the balloon reaches a preset diameter of the woven mesh, even if the balloon is further inflated, its diameter no longer changes.

13. The balloon catheter traction device as in claim 9, wherein latitudinal threads of the woven mesh consist of multiple segments of short threads, resulting in a structure of segmented woven mesh.

14. The balloon catheter traction device as in claim 13, wherein the segmented woven mesh has several complete latitudinal threads on the inner side of the balloon's anticipated or intended axial bending direction, acting as fixed-length restraints.

15. The balloon catheter traction device as in claim 9, wherein in the axial intended bending direction of the balloon, one or a nearby number of latitudinal threads of the woven mesh are of the same material as the longitudinal threads.

16. The balloon catheter traction device as in claim 1, wherein the radial restraint of the balloon is an anisotropic material, with a lower elastic modulus in the axial direction and a higher elastic modulus in the radial direction.

17. The balloon catheter traction device as in claim 1, wherein the radial restraint of the balloon is a series of radial reinforcement ribs or a reinforcement mesh applied externally to the balloon after it is formed.

18. The balloon catheter traction device as in claim 17, wherein the radial reinforcement rib or reinforcement mesh is externally coated with a polymer film or membrane for fixing the reinforcement or reinforcement mesh.

19. The balloon catheter traction device as in claim 1, wherein the radial restraint of the balloon is a woven mesh embedded within the balloon, and both the inner and outer sides of the balloon consist of thermoplastic elastomeric material and encase the woven mesh.

20. The balloon catheter traction device as in claim 1, wherein the radial restraint of the balloon is a memory metal mesh incorporated into outer sheathing of the balloon, with the memory metal mesh having a memory of a contracted or original shape.

21. The balloon catheter traction device as in claim 20, wherein the memory metal mesh has a diameter contracted to an original state.

22. The balloon catheter traction device as in claim 20, wherein the memory metal mesh of the balloon catheter traction device maintains at least one fixed-length memory metal wire on the inner side of the balloon's anticipated or intended axial bending direction.
